# EUROPÄISCHE PATENTSCHRIFT

(11) **EP 0 873 973 B1**
(45) Veröffentlichungstag und Bekanntmachung des Hinweises auf die Patenterteilung: **27.06.2001**
(21) Anmeldenummer: 98106861.2
(22) Anmeldetag: 15.04.1998
(51) Int. Cl.: C02F 1/00, E03B 11/02

(54) **Verfahren zum Befüllen eines Trinkwassertanks und Vorrichtung zur Trinkwasserversorgung**
Drinking watertank, filling process and device for drinking water supply
Procédé de remplissage d'un réservoir d'eau potable et dispositif d'allimentation en eau potable

(30) Priorität: 23.04.1997 DE 19717009
(43) Veröffentlichungstag der Anmeldung: 28.10.1998
(73) Patentinhaber: DIGMESA AG, 2563 Ipsach (CH)
(72) Erfinder: Plüss, Heinz, 3322 Schönbühl (CH)
(74) Vertreter: Lempert, Jost, Dipl.-Phys. Dr. rer.nat.

(56) Entgegenhaltungen:
- BE-A- 680 480
- GB-A- 658 020
- US-A- 4 874 496
- US-A- 5 045 195
- US-A- 5 271 837

## Beschreibung

Die Erfindung betrifft ein Verfahren zum Befüllen einer Trinkwasseranlage, die eine Frischwasserzuführleitung und einen das Trinkwasser aufnehmenden Wassertank aufweist, wobei das Wasser durch ein Filter gefiltert und durch eine Silberionenbeimischungseinheit mit Silberionen versehen wird, sowie eine Vorrichtung zur Trinkwasserversorgung aus einem Trinkwassertank mit einer an eine Frischwasserquelle anschließbaren Zuführleitung, in der sich ein Filter und eine Silberionenbeimischungseinheit befindet.

Bei mobilen bewohnbaren Einheiten, wie Schiffen, Wohnmobilen und Wohnwagen, sind ebenfalls mobile Trinkwasseranlagen vorgesehen. Diese weisen einen Wassertank auf, der an einer stationären Wasserquelle befüllt wird und dann bei Bewegung der Einheit unabhängig von stationären Wasserquellen zur Trinkwasserversorgung zur Verfügung steht. Es ist bekannt, zur Stabilisierung der Trinkwasserqualität und insbesondere zur Vermeidung von Keimen dem Wasser Silberionen zuzusetzen. Es ist weiterhin sinnvoll, das dem mobilen Tank zugeführte Wasser zu filtern, um Schadstoffe auszuscheiden, bevor das Wasser in den Tank gelangt.

Es hat sich nun herausgestellt, daß gerade ein solches Filter mit dem in ihm stehenden Restwasser in sehr hohem Maße verkeimen kann, selbst wenn dem im Wassertank selbst befindlichen Wasser Silberionen zugesetzt werden. Bei einer erneuten Befüllung wird dieses hochverkeimte Wasser aus dem Filter in den Wassertank gespült und kann auch bei dem Versehen mit Silberionen nicht in einen einwandfreien Zustand zurückgeführt werden. Es weist insbesondere einen äußerst unangenehmen Geruch auf. Dieser Mangel konnte auch nicht durch ein zusätzliches Kohleaktiv-Filter behoben werden. Andererseits ist es auch nicht empfehlenswert, die Silberionenbeimischung vor Durchfließen des Filters vorzunehmen, da die Silberionen selbst im Filter in hohem Maße ausgefiltert werden und daher die Effektivität der Silberionenbeimischung hinsichtlich der sich im im Tank befindlichen Wasser enthaltenen Silberionen sehr reduziert, also der Wirkungsgrad der Silberionenbeimischung vermindert wird.

Der Erfindung liegt daher die Aufgabe zugrunde, unter Sicherstellung eines hohen Wirkungsgrades der Silberionenbeimischung bei einer mobilen Trinkwasserversorgungsanlage ein Verkeimen des noch in der Zuleitung zum Wassertank, insbesondere auch im Filter befindlichen Wassers zu vermeiden.

Erfindungsgemäß wird die genannte Aufgabe mit einem Verfahren der eingangs genannten Art gelöst, welches dadurch gekennzeichnet ist, daß nach dem Befüllen des Tanks mit Frischwasser das Wasser zumindest über das Filter hin umgewälzt wird. Zur Lösung der Aufgabe sieht die Erfindung weiterhin eine Vorrichtung der eingangs genannten Art vor, die gekennzeichnet ist durch ein Dreiwegeventil mit einem mit dem Frischwassereinlaß verbundenen Eingang sowie einem zweiten Eingang und einer zum Tank führenden Zuführleitung, durch eine vom Wassertank zum zweiten Einlaß des Dreiwegeventils führende Rückleitung und durch eine Umwälzpumpe im durch Rück- und Zuführleitung gebildeten Wasserkreislauf.

Dadurch, daß nach dem Befüllen das im Tank befindliche und mit Silberionen versehene Frischwasser noch einmal durch das gesamte Leitungssystem und insbesondere auch das Filter umgewälzt wird, wird erreicht, daß im gesamten Leitungssystem und insbesondere auch im Filter mit Silberionen versehenes Wasser steht und damit zusätzlich zum Tank auch das gesamte Leitungssystem, einschließlich des Filters, vor einem Verkeimen geschützt ist. Obwohl auch bei der erfindungsgemäßen Ausgestaltung der Vorrichtung die Silberionenbeimischungseinheit grundsätzlich vor dem Filter angeordnet werden könnte, wird sie in der Regel zur Erhöhung bzw. Sicherung der Effektivität der Silberionenbeimischung in Durchflußrichtung hinter dem Filter bzw. stromab desselben und vorzugsweise unmittelbar vor dem Tank angeordnet, so daß gemäß einer bevorzugten Weiterbildung des erfindungsgemäßen Verfahrens vorgesehen ist, daß das umgewälzte Wasser zuerst durch das Filter und dann die Silberionenbeimischungseinheit gepumpt wird.

Das Verfahren kann in bevorzugter Weise dadurch weitergebildet sein, daß das Wasser des Wassertanks über ein Dreiwegeventil in die Frischwasserzuführleitung geführt wird und daß ein in der Zuführleitung befindliches Dreiwegeventil nach Befüllen des Wassertankes von einem mit einer Frischwasserleitung verbindbaren Anschluß zu einer vom Wassertank zum Dreiwegeventil führenden Umwälzleitung umgeschaltet und eine Umwälzpumpe in Betrieb gesetzt wird.

Hierdurch wird nach dem Befüllen des Tanks und Erreichen des Maximalfüllstandes ein automatisches Umwälzen bewirkt, so daß sichergestellt ist, daß immer und in jedem Fall nach dem Befüllen ein Umwälzen erfolgt, ohne daß zusätzliche Bedienungsvorgänge erforderlich sind, die vergessen werden könnten.

Die erfindungsgemäße Vorrichtung sieht in Weiterbildung vor, daß die Pumpe sich in der Zuführleitung befindet. Eine weitere bevorzugte Ausgestaltung sieht vor, daß in der die Silberionenbeimischungseinheit aufweisenden Leitung ein Durchflußmeßgerät angeordnet ist, mittels dessen die Silberionenabgaberate der Silberionenbeimischungseinheit in Abhängigkeit von der Durchflußrate steuerbar ist. Ein Überfüllen des Tanks bzw. eine Entleerung ohne vorherige Warnung kann dadurch verhindert werden, daß der Wassertank mit Niveausensoren versehen ist. Bei Unterschreiten eines vorgegebenen Mindestniveaus kann ein entsprechender Niveausensor ein Warnsignal abgeben; bei Erreichen des Maximalniveaus kann ein automatisches Trennen der erfindungsgemäßen Vorrichtung von der (stationären) Wasserversorgung erfolgen, indem der Einlaß des Dreiwegeventils zur stationären Wasserversorgung geschlossen und gegebenenfalls gleichzeitig die Umschaltung zur Rücklaufleitung vorgenommen wird, also das Öffnen des mit dieser verbundenen Einlasses des Dreiwegeventils. Grundsätzlich kann das Befüllen auch vor Erreichen des Maximalfüllstandes beendet werden. Auch in diesem Falle wird das Dreiwegeventil zum Umwälzen umgeschaltet. In äußerst bevorzugter Ausgestaltung ist hierzu ein Steuergerät zum Umschalten des Dreiwegeventils und gegebenenfalls anschließenden Einschalten der Pumpe nach Erreichen des maximalen Füllstandes im Wassertank vorgesehen. Für das Umwälzen reicht eine sehr kurze Zeit, beispielsweise weniger als 60 sec. Durch die Erfindung wird erreicht, daß die gesamte Anlage bzw. die wasserführenden Teile mit mit Silberionen behandeltem Wasser gefüllt sind und vermieden wird, daß das Wasser verkeimt.

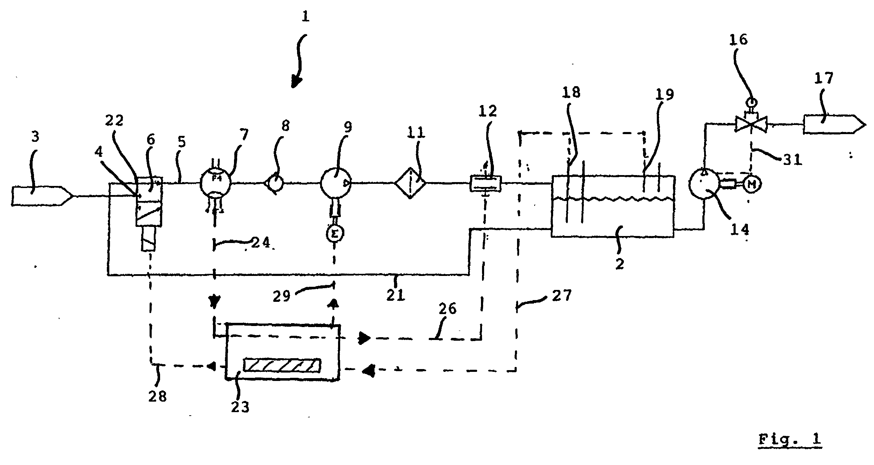

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnung im einzelnen erläutert ist. Dabei zeigt die einzige Figur:
das bevorzugte Ausführungsbeispiel der erfindungsgemäßen Vorrichtung in schematischer Darstellung.

Die erfindungsgemäße Vorrichtung 1 zur Trinkwasserversorgung aus einem Trinkwassertank 2 weist zunächst diesen Trinkwassertank 2 auf. Dem Trinkwassertank 2 wird Frischwasser über einen Trinkwassereinlaß 3, den Eingang 4 eines Dreiwegeventils 6 und eine Zuführleitung 5 zugeführt, in der die folgenden weiteren Teile in der angegebenen Reihenfolge angeordnet sind: Durchflußmesser 7, Rückschlagventil 8, Umwälzpumpe 9, Filter 11, Silberionenbeimischungseinheit 12. Von dem Trinkwassertank 2 führt eine Auslaßleitung 13 über eine Entnahmepumpe 14 und einen Entnahme-Wasserhahn 16 zu einem Wasserauslaß 17. Im Tank 2 befinden sich zwei Niveausensoren 18, 19 zum Feststellen des maximalen und des minimalen Füllstandes im Wassertank 2.

Vom Wassertank 2 führt eine Rückleitung 21 zu einem zweiten Eingang 22 des Dreiwegeventils 6. Die erfindungsgemäße Vorrichtung 1 weist weiterhin ein Steuergerät 23 auf. Von dem Durchflußmesser 7 führt eine Durchfluß-Meßleitung 24 zum Steuergerät 23 und von diesem eine StromSteuerleitung 26 zur Silberionenbeimischungseinheit 12. Weiter führt eine Niveaumeßleitung 27 von den Niveausensoren 18, 19 zum Steuergerät 23. Vom Steuergerät 23 führen eine Ventil-Steuerleitung 28 und eine Pump-Steuerleitung 29 zum Dreiwegeventil 6 bzw. der Pumpe 9. Schließlich ist noch eine Entnahmepumpen-Steuerleitung 31 vom Entnahme-Wasserhahn 16 zur Entnahme-Pumpe 14 geführt; diese kann ebenfalls über das Steuergerät 23 geführt sein.

Zum Befüllen des Wassertanks 2 erfolgt der Anschluß der erfindungsgemäßen Vorrichtung über den Frischwassereinlaß 4 an eine stationäre Frischwasserversorgungsanlage. Unter dem Eigendruck der Frischwasserversorgungsanlage, wobei dann die Umwälzpumpe 9 im Leerlauf läuft, oder aber auch bei aktivierter Umwälzpumpe 9 wird Frischwasser durch die Zuführleitung 5 und die in ihr angeordneten Elemente dem Frischwassertank 2 zugeführt, bis der Maximal-Niveausensor 19 ein entsprechendes Füllsignal über die Niveau-Meßleitung 27 an das Steuergerät 23 abgibt. Dieses schließt daraufhin den Eingang 4 des Dreiwegeventils 6 und öffnet den Eingang 22 über die Steuerleitung 28 und stellt gegebenenfalls über die Steuerleitung 29 die Pumpe 9 an. Diese wälzt dann das im Tank 2 und dem Leitungssystem befindliche Wasser durch das Leitungssystem um. Während des Durchflusses von Wasser durch die Leitung 5 und damit den Durchflußmesser 7, und zwar unabhängig davon, ob Frischwasser zugeführt wird oder Wasser in der vorstehend beschriebenen Weise umgewälzt wird, mißt der Durchflußmesser 7 den Durchfluß und gibt ein entsprechendes Durchflußmeßsignal über die Meßleitung 24 ab. In Abhängigkeit von dem Durchflußmeßsignal wird die Silberionenbeimischungseinheit 12 über die Steuerleitung 26 mit einem entsprechend an den Wasserdurchfluß angepaßten Strom beaufschlagt, wodurch Silberionen aus der Silberionenbeimischungseinheit 12 in das durchfließende Wasser durchflußabhängig und damit genau dosiert abgegeben werden. Um einen regelmäßigen Abtrag der in dieser Einheit befindlichen Silberplättchen zu erreichen, wird in gewissen Abständen durch die Steuereinheit 23 eine Umpolung der Elektroden vorgenommen. Das Umwälzen des Wassers erfolgt über eine vorgegebene kurze Zeit im Bereich von 30 bis 60 sec.

Durch die Erfindung wird erreicht, daß das gesamte in der Wasserversorgungsvorrichtung 1 befindliche Wasser und nicht nur das Wasser im Tank selbst, sondern insbesondere das Wasser auch in der Zuführleitung 5 und vor allem im Filter 11 mit Silberionen behandelt ist, wodurch eine Verkeimung des Wassers insbesondere auch im Filter 11 vermieden wird.

Zur Entnahme von Wasser aus dem Wassertank 2 wird der Hahn 16 geöffnet, der die Entnahmepumpe 14 in Betrieb setzt, welche aus dem Wassertank 2 Wasser abpumpt und zum Auslaß 17 führt. Wenn der Wasserspiegel im Wassertank 2 so weit absinkt, daß der Niveausensor 18 anspricht, so gibt dieser ein entsprechendes Signal an die Steuereinheit 3 ab, die daraufhin ein akustisches und/oder optisches Warnsignal erzeugt, womit die Notwendigkeit des Nachfüllens des Wassertanks 2 angezeigt wird.

## Patentansprüche

1. Verfahren zum Befüllen einer Trinkwasseranlage, die eine Frischwasserzuführleitung und einen das Trinkwasser aufnehmenden Wassertank aufweist, wobei das Wasser durch ein Filter gefiltert und durch eine Silberionenbeimischungseinheit mit Silberionen versehen wird, dadurch gekennzeichnet, daß nach dem Befüllen des Tanks mit Frischwasser das Wasser zumindest über das Filter hin umgewälzt wird.

2. Verfahren nach Anspruch 1, dadurch gekennzeichnet, daß das umgewälzte Wasser zuerst durch das Filter und dann die Silberionenbeimischungseinheit gepumpt wird.

3. Verfahren nach Anspruch 1 oder 2, dadurch gekennzeichnet, daß das Wasser des Wassertanks über ein Dreiwegeventil in die Frischwasserzuführleitung geführt wird.

4. Verfahren nach einem der vorangehenden Ansprüche, dadurch gekennzeichnet, daß ein in der Zuführleitung befindliches Dreiwegeventil nach Befüllen des Wassertankes von einem mit einer Frischwasserleitung verbindbaren Anschluß zu einer vom Wassertank zum Dreiwegeventil führenden Umwälzleitung umgeschaltet und eine Umwälzpumpe in Betrieb gesetzt wird.

5. Verfahren nach einem der vorangehenden Ansprüche, dadurch gekennzeichnet, daß das Umwälzen des im Wassertank befindlichen Wassers nach einer vorgegebenen Zeit bewirkt wird.

6. Vorrichtung zur Trinkwasserversorgung aus einem Trinkwassertank (2) mit einer an eine Frischwasserquelle anschließbaren Zuführleitung (5), in der sich ein Filter (11) und eine Silberionenbeimischungseinheit (12) befindet, gekennzeichnet durch ein Dreiwegeventil (6) mit einem mit dem Frischwassereinlaß (3) verbundenen Eingang (4) sowie einem zweiten Eingang (22) und einer zum Tank (2) führenden Zuführleitung (5), durch eine vom Wassertank (2) zum zweiten Einlaß (22) des Dreiwegeventils (6) führende Rückleitung (21) und durch eine Umwälzpumpe (9) im durch Rück- und Zuführleitung (21, 5) gebildeten Wasserkreislauf.

7. Vorrichtung nach Anspruch 5, dadurch gekennzeichnet, daß die Pumpe (9) sich in der Zuführleitung (5) befindet.

8. Vorrichtung nach Anspruch 6 oder 7, dadurch gekennzeichnet, daß die Silberionenbeimischungseinheit (12) sich stromab des Filters (11) befindet.

9. Vorrichtung nach einem der Ansprüche 6 bis 8, dadurch gekennzeichnet, daß in der die Silberionenbeimischungseinheit (12) aufweisenden Leitung (5) ein Durchflußmeßgerät angeordnet ist, mittels dessen die Silberionenabgaberate der Silberionenbeimischungseinheit (12) in Abhängigkeit von der Durchflußrate steuerbar ist.

10. Vorrichtung nach einem der Ansprüche 6 bis 9, dadurch gekennzeichnet, daß der Wassertank (2) mit Niveausensoren (18, 19) versehen ist.

11. Vorrichtung nach einem der Ansprüche 6 bis 10, gekennzeichnet durch ein Steuergerät (23) zum Umschalten des Dreiwegeventils (6) und gegebenenfalls anschließenden Einschalten der Pumpe (9) nach Erreichen des maximalen Füllstandes im Wassertank (2).

## Claims

1. Method for filling a drinking water installation having a fresh water supply pipe and a water tank receiving the drinking water, in which the water is filtered by a filter and provided with silver ions by a silver ion admixing unit, characterized in that, after filling the tank with fresh water, the water is at least circulated over the filter.

2. Method according to claim 1, characterized in that the circulated water is firstly pumped through the filter and then the silver ion admixing unit.

3. Method according to claim 1 or 2, characterized in that the water from the water tank is passed by means of a three-way valve into the fresh water supply pipe.

4. Method according to one of the preceding claims, characterized in that, after filling the water tank, a three-way valve in the supply pipe is switched over from a connection connectable with a fresh water pipe to a circulating pipe leading from the water tank to the three-way valve and a circulating pump is put into operation.

5. Method according to one of the preceding claims, characterized in that the circulation of the water in the water tank is brought about after a predetermined time.

6. Apparatus for drinking water supply from a drinking tank (2) with a supply pipe (5) connectable to a fresh water source and in which is located a filter (11) and a silver ion admixing unit (12), characterized by a three-way valve (6) with an inlet (4) connected to the fresh water intake (3), as well as a second inlet (22) and a supply pipe (5) leading to the tank (2), by a return pipe (21) leading from the water tank (2) to the second inlet (22) of the three-way valve (6) and by a circulating pump (9) in the water circuit formed by the return pipe (21) and supply pipe (5).

7. Apparatus according to claim 5, characterized in that the pump (9) is in the supply pipe (5).

8. Apparatus according to claim 6 or 7, characterized in that the silver ion admixing unit (12) is located downstream of the filter (11).

9. Apparatus according to one of the claims 6 to 8, characterized in that the pipe (5) containing the silver ion admixing unit (12) is provided with a flowmeter by means of which it is possible to control the silver ion delivery rate of the silver ion admixing unit (12) as a function of the flow rate.

10. Apparatus according to one of the claims 6 to 9, characterized in that the water tank (2) is provided with level sensors (18, 19).

11. Apparatus according to one of the claims 6 to 10, characterized by a control device (23) for switching over the three-way valve (6) and optionally subsequent connecting in of the pump (9) on reaching the maximum filling level in the water tank (2).

## Revendications

1. Procédé de remplissage d'une installation d'eau potable présentant une conduite d'amenée d'eau douce et un réservoir recevant l'eau potable, dans lequel on filtre l'eau à travers un filtre et on lui apporte des ions d'argent par l'intermédiaire d'une unité d'apport d'ions d'argent, caractérisé en ce qu'après remplissage du réservoir en eau douce, on fait circuler l'eau au moins à travers le filtre.

2. Procédé selon la revendication 1, caractérisé en ce que l'eau que l'on fait circuler est d'abord pompée à travers le filtre et ensuite à travers l'unité d'apport d'ions d'argent.

3. Procédé selon la revendication 1 ou 2, caractérisé en ce que l'eau du réservoir est amenée dans le conduit d'amenée de l'eau douce au moyen d'une vanne à trois voies.

4. Procédé selon l'une quelconque des revendications précédentes, caractérisé en ce qu'après remplissage du réservoir, une vanne à trois voies disposée dans le conduit d'amenée est commutée d'une jonction avec raccord reliable à un conduit d'eau douce vers un conduit de mise en circulation conduisant du réservoir à la vanne à trois voies et en ce qu'une pompe de circulation est mise en service.

5. Procédé selon l'une quelconque des revendications précédentes, caractérisé en ce que la mise en circulation de l'eau se trouvant dans le réservoir a lieu après une durée prédéterminée.

6. Dispositif d'alimentation en eau potable consistant en un réservoir (2) d'eau potable présentant un conduit d'amenée (5) pouvant être raccordé à une source d'eau douce, dans lequel sont disposés un filtre (11) et une unité d'apport d'ions d'argent (12), caractérisé par une vanne à trois voies (6) présentant une entrée (4) reliée à l'amenée d'eau douce (3) ainsi qu'une deuxième entrée (22) et un conduit d'amenée (5) menant au réservoir (2), et caractérisé par un conduit de retour (21) menant du réservoir (2) à la deuxième entrée (22) de la soupape à trois voies (6) et par une pompe de circulation (9) dans le circuit d'eau constitué par les conduits de retour et d'amenée (21,5).

7. Dispositif selon la revendication 5, caractérisé en ce que la pompe (9) est disposée dans le conduit d'amenée (5).

8. Dispositif selon la revendication 6 ou 7, caractérisé en ce que l'unité d'apport d'ions d'argent (12) se trouve en aval du filtre (11).

9. Dispositif selon l'une quelconque des revendications 6 à 8, caractérisé en ce que dans le conduit (5) présentant l'unité d'apport d'ions d'argent est disposé un instrument de mesure de débit, permettant de commander le taux de libération d'ions d'argent par l'unité d'apport d'ions d'argent (12) en fonction de la valeur du débit.

10. Dispositif selon l'une quelconque des revendications 6 à 9, caractérisé en ce que le réservoir (2) est équipé de détecteurs de niveau (18,19).

11. Dispositif selon l'une quelconque des revendications 6 à 10, caractérisé par un organe de commande (23) pour la commutation de la vanne à trois voies (6) et le cas échéant la mise en marche consécutive de la pompe (9) lorsque le niveau de remplissage maximal du réservoir (2) est atteint.
